# EUROPEAN PATENT SPECIFICATION

(11) **EP 3 275 164 B1**
(45) Date of publication and mention of the grant of the patent: **24.06.2020**
(21) Application number: 16715809.6
(22) Date of filing: 24.03.2016
(51) Int. Cl.: H04M 1/725, A61B 5/00, G06F 1/16, H04W 4/00, G04G 21/00, H04W 4/38, H04W 4/029, G06F 3/01

(54) **LEARNING MODE FOR CONTEXT IDENTIFICATION**
LERNMODUS ZUR KONTEXTIDENTIFIZIERUNG
MODE D'APPRENTISSAGE POUR IDENTIFICATION DE CONTEXTE

(30) Priority: 24.03.2015 US 201562137712 P; 23.07.2015 EP 15178057
(43) Date of publication of application: 31.01.2018
(73) Proprietor: Koninklijke Philips N.V., 5656 AG Eindhoven (NL)
(72) Inventor: CRONIN, John, 5656 AE Eindhoven (NL); CRONIN, Seth, 5656 AE Eindhoven (NL)
(74) Representative: van Velzen, Maaike Mathilde
(86) International application number: PCT/EP2016/056614
(87) International publication number: WO 2016/151099

(56) References cited:
- EP-A1- 2 608 503
- US-A1- 2013 303 198

## Description

### TECHNICAL FIELD

This disclosure concerns wearable technology. More particularly, but not exclusively, the present disclosure concerns wearable devices that over time learn to identify the contexts in which they are being used.

### BACKGROUND

Wearable technology may include any type of mobile electronic device that can be worn on the body, attached to or embedded in clothes and accessories of an individual, and currently exists in the consumer marketplace. Processors and sensors associated with the wearable technology can display, process, or gather information. Such wearable technology has been used in a variety of areas, including monitoring health data of the user as well as other types of data and statistics. These types of devices may be readily available to the public and may be easily purchased by consumers. Examples of some wearable technology in the health arena include the FitBit Flex™, the Nike Fuel Band™, the Jawbone Up™, and the Apple Watch™ devices. EP 2 608 503 discloses a method and mobile device for determining a state of the mobile device.

### SUMMARY

Wearable devices that predict the context in which they are used based on previously tracked context data, and methods associated with the same, are provided. The invention is defined in and by the appended claims.

Various examples described herein are directed to a wearable device comprising: a sensor configured to obtain sensor data descriptive of a physiological parameter of the user; a memory configured to store a plurality of records correlating context data to physiological parameters obtained from sensor data; and a processor configured to:
establish a baseline value based on a first set of sensor data obtained from the sensor and retrieved from memory (115),
compare the second set of sensor data obtained from the sensor to the baseline value to determine whether the second set of sensor data is a change from the baseline value, request user input from the user descriptive of a current context in response to determining that the second set of sensor data is a change from the baseline value, create a record based on the second set of sensor data and the current context, and store the record in the memory as a member of the plurality of records.

Various examples described herein are directed to a method for training a wearable device to predict a context in which a wearable device is used, the method comprising: establishing a baseline value based on a first set of sensor data obtained from a sensor configured to obtain sensor data descriptive of a physiological parameter of the user, comparing the baseline value to a second set of sensor data obtained from the sensor to determine whether the second set of sensor data is a change from the first set of sensor data, requesting user input from the user descriptive of a current context in response to determining that the second set of sensor data is a change from the first set of sensor data, creating a record based on the second set of sensor data and the current context, and storing the record in a memory as a member of a plurality of records.

Various examples described herein are directed to a non-transitory computer-readable medium having a computer program stored thereon, the computer program executable by a processor to perform a method for predicting a context in which a wearable health device is used, the method comprising: instructions for establishing a baseline value based on a first set of sensor data obtained from a sensor configured to obtain sensor data descriptive of a physiological parameter of the user; instructions for comparing the baseline value to a second set of sensor data obtained from the sensor to determine whether the second set of sensor data is a change from the first set of sensor data; instructions for requesting user input from the user descriptive of a current context in response to determining that the second set of sensor data is a change from the first set of sensor data; instructions for creating a record based on the second set of sensor data and the current context; and instructions for storing the record in a memory as a member of a plurality of records.

The method, device and the non-transitory machine readable medium as described above provides an improved method of constructing a labeled set of data for use in future determinations of user context (*e.g.,* activity or emotional state). By identifying deviations from baseline physiological parameters, opportunities for requesting user input for labeling training examples for inclusion in a training set can be easily identified. The training set can then be used to train a machine learning algorithm (or otherwise used) for determining user context in the future from similar readings without user input. This approach is an improvement over relying on the user to identify such opportunities which may be unreliable, inconsistent, and overly-burdensome on the user. The approach is particularly lightweight when compared to training and employing additional trained models (*e.g.,* logistic regression) for the additional purpose of identifying such key moments which may not be practicable in situations where processing power is limited (*e.g.* as is the case in many wearable devices).

Various examples additionally include a communication interface configured to communicate over a communication network, wherein the communication interface is configured to detect the presence of one or more other wearable devices communicatively coupled to the network.

Various examples are described wherein the communication interface is further configured to receive additional context data from the one or more other wearable devices communicatively coupled to the network, and wherein the record includes the additional context data.

Various examples are described wherein the processor is further configured to: train a machine-learning model using the plurality of records; and apply the machine-learning model to a third set of sensor data obtained from the sensor at a later time to estimate a context at the later time.

Various examples are described establishing the baseline value comprises obtaining a statistical mode figure from the first set of sensor data.

### BRIEF DESCRIPTION OF THE DRAWINGS

FIG. 1 illustrates an exemplary wearable device.
FIG. 2 illustrates an exemplary settings GUI that may be rendered and displayed on a display of a wearable device.
FIG. 3 illustrates an exemplary learn mode GUI that may be rendered and displayed on a display of a wearable device.
FIG. 4 illustrates an exemplary context GUI that may be rendered and displayed on a display of a wearable device.
FIG. 5 illustrates an exemplary geo GUI that may be rendered and displayed on a display of a wearable device.
FIG. 6 illustrates an exemplary computing device architecture.
FIG. 7 illustrates an exemplary analysis GUI that may be rendered and displayed on a display of a wearable device.
FIG. 8 illustrates an exemplary operational process performed by a base software module stored in memory and executed by a processor of a wearable device.
FIG. 9 illustrates an exemplary operational process performed by a context learn software module stored in memory and executed by a processor of a wearable device.
FIG. 10 illustrates an exemplary baseline subroutine performed by a context learn software module stored in memory and executed by a processor of a wearable device.
FIG. 11 illustrates an exemplary operational process performed by a predict context software module stored in memory and executed by a processor of a wearable device.
FIG. 12 illustrates an exemplary operational process performed by an analysis software module stored in memory and executed by a processor of a wearable device.
FIG. 13 illustrates an exemplary method for predicting a context in which a wearable health device is used by a user based on contextual information previously supplied by the user.

### DETAILED DESCRIPTION

The description and drawings presented herein illustrate various principles. It will be appreciated that those skilled in the art will be able to devise various arrangements that, although not explicitly described or shown herein, embody these principles and are included within the scope of this disclosure. As used herein, the term, "or," as used herein, refers to a non-exclusive or (i.e., and/or), unless otherwise indicated (e.g., "or else" or "or in the alternative"). Additionally, the various embodiments described herein are not necessarily mutually exclusive and may be combined to produce additional embodiments that incorporate the principles described herein.

Although existing wearable devices are useful in some regards, their usefulness may be limited by their inability to predict the context in which they are being used. As a result, wearable devices miss out on opportunities to enhance wearable sensor data based on contextual information. Current wearable devices do not have the ability to track contextual input over time and use the collected data to later predict contexts without requiring user input. Given those shortcomings, it would be desirable for a smart wearable device to, over time, learn to identify the contexts in which it is used.

In view of the foregoing, wearable devices that predict the context in which they are used based on previously tracked user input, and methods associated with the same, are provided. The wearable device may be any kind of wearable device, such as one primarily intended to be worn around a user's neck (*e.g.,* a necklace), arm (*e.g.,* an armband), head (*e.g.,* hat, helmet, headband, or headlamp), leg, chest, or waist (*e.g.,* a belt or body band), foot (*e.g.,* a shoe or sock), ankle or knee (*e.g.,* a knee brace), or any other area of a user's body. The wearable device may also be a device that is primarily intended to be held in a user's hand or stored in a user's pocket. The wearable device may also be a device meant to be worn over the skin (*e.g.,* a patch or garment) or under the skin (*e.g.,* an implant).

The wearable devices (*e.g*., an Apple Watch™ device) may include, as implemented in various systems, methods, and non-transitory computer-readable storage media, a context learn mode that a user of the wearable device may activate. In some embodiments, the context learn mode may be active by default. The context learn mode may identify a context in which the wearable device records data associated with a user of the wearable device (*e.g.,* health-related data). When in context learn mode, the wearable device may detect changes in wearable sensor data and prompt the user to input contextual information (*e.g.,* an emotion felt by the user when the wearable device is being used). Using the context learn mode, the wearable device may track user input over time and then use the tracked input to later predict-without any additional then-current contextual information provided by the user-the context in which the wearable device is then being used. The wearable device may track user input and correlate input with sensor data.

In one exemplary scenario, for instance, a wearable device user may produce a change in health-related sensor data (*e.g.,* a change in motion, heart rate, blood pressure, temperature, geolocation, or the like being tracked by the wearable device by way of one or more sensors). The change in sensor data may occur as the user experiences a particular emotion, participates in a particular activity, a combination of both events, or as a result of other environmental influences. Using the context learn mode, the user may provide contextual input that correlates the change in sensor data with the activity, emotion, or other influence. The wearable device may receive the contextual input from the user in a variety of ways. The wearable device may, for instance, receive a "tag" by which a user tags a particular change in sensor data with an activity, emotion, or other influence. The next time the wearable device detects a similar change in sensor data, the wearable device may predict that the user is experiencing the same activity, emotion, or other influence that the user experienced when the user previously inputted the contextual information (*e.g.,* tagged the sensor data with the activity, emotion, or other influence). Over time, the more contextual information the wearable device receives from the user, the better the wearable device can predict contexts without the need for concurrent contextual information supplied by the user. By learning to independently and automatically identify the context in which it is being used, the wearable device may provide more contextually relevant information to the user. As a result, the user's perspective on the data may be enhanced. The user may, in effect, be equipped with a greater ability to understand the impact of certain activities, emotions, or other influences on his or her body.

The context learn mode may increase the usefulness of wearable device data by expanding the number of ways in which a user might use wearable device sensor data. As discussed above, the context learn mode may allow a user to infuse the data with relevant context. Equipped with the context learn mode, a wearable device may show a user's body output based on the user's activities or emotions. Over time, the context learn mode may also improve the wearable device experience by demanding less and less input from the user while at the same time providing more and more useful data based on predicted contexts. The predicted contexts may be based on input the user has already supplied in the past. For example, the user input may be used as a label for a training example such as, for example, a record including the present or recent sensor data (*e.g.,* raw sensor data or features extracted from raw sensor data such as, for example, a heart rate extracted from raw optic data from an optical sensor or a location tag associated with GPS location data) along with the user-input label(s). Thereafter, a collection of such training examples ("training set") may be used to train one or more machine-learning models (*e.g.,* logistic regression or neural networks using gradient descent) to identify the context tags from present or recent sensor data at a time in the future.

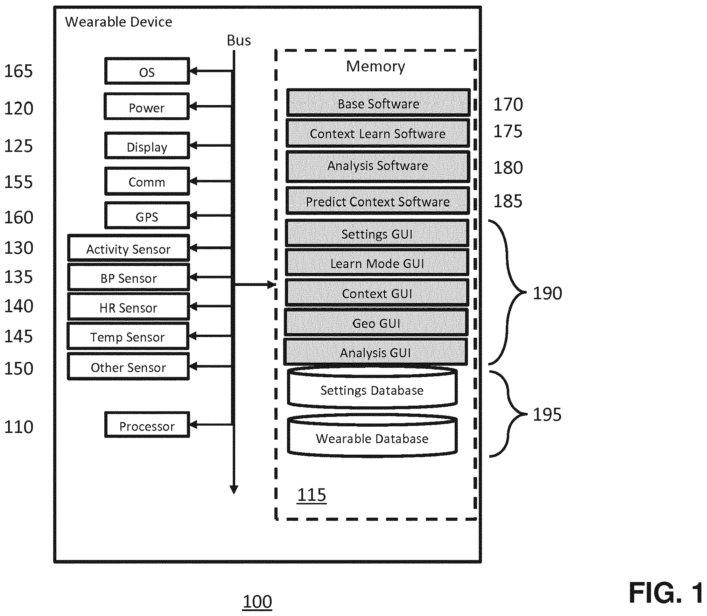

FIG. 1 illustrates an exemplary wearable device 100. Wearable device 100 may include a plurality of components. In some embodiments, the components may be connected by a single bus 105, as illustrated in FIG. 1. In other embodiments, the components may be connected through multiple buses 105. The plurality of components may include a processor 110, memory 115, a power supply 120 (*e.g.,* a rechargeable lithium ion battery), a display 125 (*e.g.,* an LCD, LED, e-paper, or electronic ink type display), one or more sensors (*e.g.,* an activity sensor 130, a blood pressure sensor 135, a heart rate sensor 140, a temperature sensor 145, or other sensor 150), and a wired or wireless network communications module 155 (*e.g.,* a USB port module, a FireWire port module, a Lightning port module, a Thunderbolt port module, a Wi-Fi connection module, a 3G/4G/LTE cellular connected module, a Bluetooth connection module, a lower powered Bluetooth connection module, a ZigBee module, a near field communication module, etc.). The components may further include a global position system ("GPS") module 160.

While an example set of sensors 130-150 are illustrated, it will be apparent that various embodiments may utilize sets that include fewer, additional, or alternative sensors. The sensor devices 110 may be virtually any sensor capable of sensing data about a user, the user's environment, the user's context, the state of various electronics associated with the user, etc. In some embodiments, the sensor devices 110 may sense physiological parameters about the user. For example, the sensor devices 110 may include accelerometers, conductance sensors, optical sensors, temperature sensors, microphones, cameras, etc. These or other sensors may be useful for sensing, computing, estimating, or otherwise acquiring physiological parameters descriptive of the wearer such as, for example, steps taken, walking/running distance, standing hours, heart rate, respiratory rate, blood pressure, stress level, body temperature, calories burned, resting energy expenditure, active energy expenditure, height, weight, sleep metrics, etc.

Wearable device 100 may be operable to store in memory 115, and processor 110 of wearable device 100 may be operable to execute, a wearable device operating system ("OS") 165 and a plurality of executable software modules. As used herein, the term "processor" will be understood to encompass various hardware devices such as, for example, microprocessors, field-programmable gate arrays (FPGAs), application specific integrated circuits (ASICs), and other hardware devices capable of performing the various functions described herein as being performed by the wearable 100 or other device. Further, the memory 115 may include various devices such as L1/L2/L3 cache, system memory, or storage devices and, while not shown, some of the components of the server (*e.g.,* components 170-195) will also be understood to be stored among one or more similar memory devices. As used herein, the term "non-transitory machine-readable storage medium" will be understood to refer to both volatile memory (*e.g.,* SRAM and DRAM) and non-volatile memory (*e.g.,* flash, magnetic, and optical memory) devices, but to exclude mere transitory signals. While various embodiments may be described herein with respect to software or instructions "performing" various functions, it will be understood that such functions will actually be performed by hardware devices such as a processor executing the software or instructions in question. In some embodiments, such as embodiments utilizing one or more ASICs, various functions described herein may be hardwired into the hardware operation; in such embodiments, the software or instructions corresponding to such functionality may be omitted.

The OS may be, for example, a Microsoft Windows™, Google Android™, or Apple™ OS. The executable software modules may include a base software module 170, a context learn software module 175, an analysis software module 180, a predict context software module 185, and one or more graphical user interface ("GUI") software modules 190 that, when executed, render and display one or more GUIs on a display of the wearable device (*e.g.,* a settings GUI module, a learn mode GUI module, a context GUI module, a geo GUI module, an analysis GUI module, etc.). The wearable device may further be operable to store one or more databases 195 in memory (*e.g.,* a settings database, a wearable database, etc.).

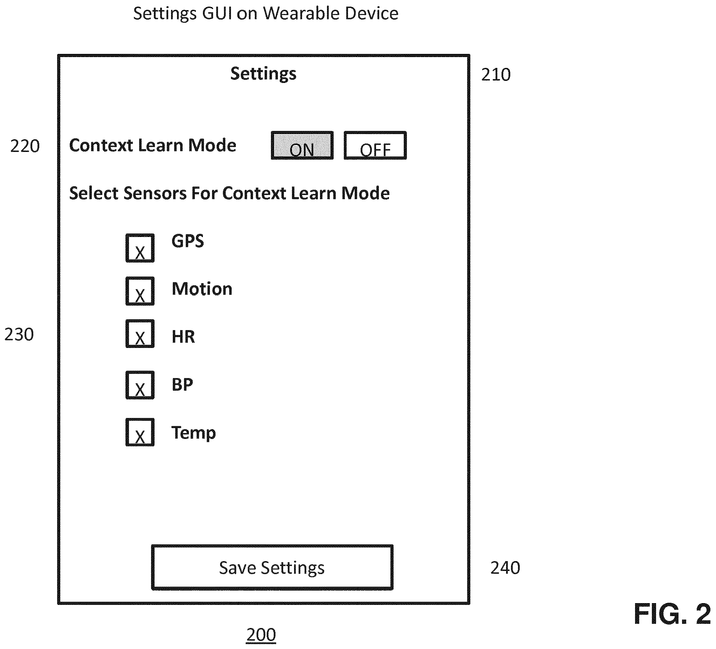

FIG. 2 illustrates an exemplary settings GUI 200 that may be rendered and displayed on a display of a wearable device. Settings GUI 200 may include a header 210 with a title identifying it as the settings GUI. Settings GUI 200 may include a selectable element 220 (*e.g.,* a button, switch, toggle, or the like) that, when selected by a user of the wearable device, may cause the wearable device to turn context learn mode on or off. As shown in FIG. 2, context mode is turned on. Settings GUI 200 may further include one or more selectable elements 230 that, when selected by the user, may allow the user to select one or more sensors to which context learn mode will be applied when turned on. As shown in FIG. 2, exemplary sensors include a GPS sensor, a motion sensor, a heart rate sensor, a blood pressure sensor, and a temperature sensor (all of which are selected in the exemplary GUI provided for illustrative purposes). Settings GUI 200 may contain a selectable element 240 that, when selected by the user, may cause the wearable device to store the user-configured settings to a settings database stored in memory of the wearable device. The settings database may alternatively be stored in a separate and distinct computing device (*e.g.,* a database server) communicatively coupled to the wearable device.

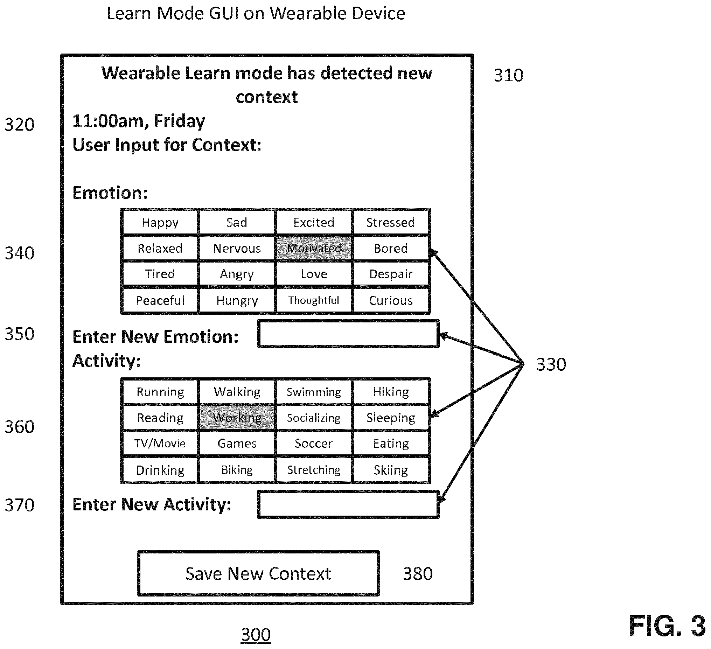

FIG. 3 illustrates an exemplary learn mode GUI 300 that may be rendered and displayed on a display of a wearable device. Learn mode GUI 300 may include a header 310 with a message identifying it as the learn mode GUI. As shown in FIG. 3, the title may read "Wearable learn mode has detected new context." Header 300 may include additional information 320 about the detected context, such as the time and day at which it was detected (*e.g.,* 11:00 AM on Friday). Learn mode GUI 300 may further include one or more fillable or selectable fields 330 that may receive context data inputted by a user of the wearable device. Learn mode GUI 300 may request that the user submit context data via fillable or selectable fields 330. In one embodiment, one type of context data may include emotion. In such an embodiment, learn mode GUI 300 may include a grid of selectable elements 340 (*e.g.,* buttons) that, when selected by the user, submit context data to the wearable device (*e.g*., the specification of a particular emotion associated with the current context at a given time and day). The grid may include, for example, emotions such as happy, sad, excited, stressed, relaxed, nervous, motivated (which is selected in the example shown in FIG. 3), bored, tired, angry, love, despair, peaceful, hungry, thoughtful, curious, and other emotions. Learn mode GUI 300 may further include a fillable form 350 through which a user may submit, and the wearable device may receive, custom context data (*e.g.,* a custom emotion not shown on the provided grid or otherwise displayed as a predetermined selectable data submission option).

Learn mode GUI 300 may further include selectable elements 360 (*e.g.,* a grid of selectable buttons or a free fillable form) directed to other types of context data, such as an activity. As shown in FIG. 3, selectable elements 360 may be arranged in a grid and may include activities such as running, walking, swimming, hiking, reading, working (which is selected in the example shown in FIG. 3), socializing, sleeping, watching TV/movie, games, soccer, eating, drinking, biking, stretching, skiing, and other emotions. Learn mode GUI 300 may further include a fillable form 370 through which a user may submit, and the wearable device may receive, custom context data (*e.g.,* a custom activity not shown on the provided grid or otherwise displayed as a predetermined selectable data submission option). Learn mode GUI 300 may contain a selectable element 380 that, when selected by the user, may cause the wearable device to improve the context learning capabilities of the wearable device by storing the user-supplied context data to a database stored in memory of the wearable device. The database may alternatively be stored in a separate and distinct computing device (*e.g.,* a database server) communicatively coupled to the wearable device.

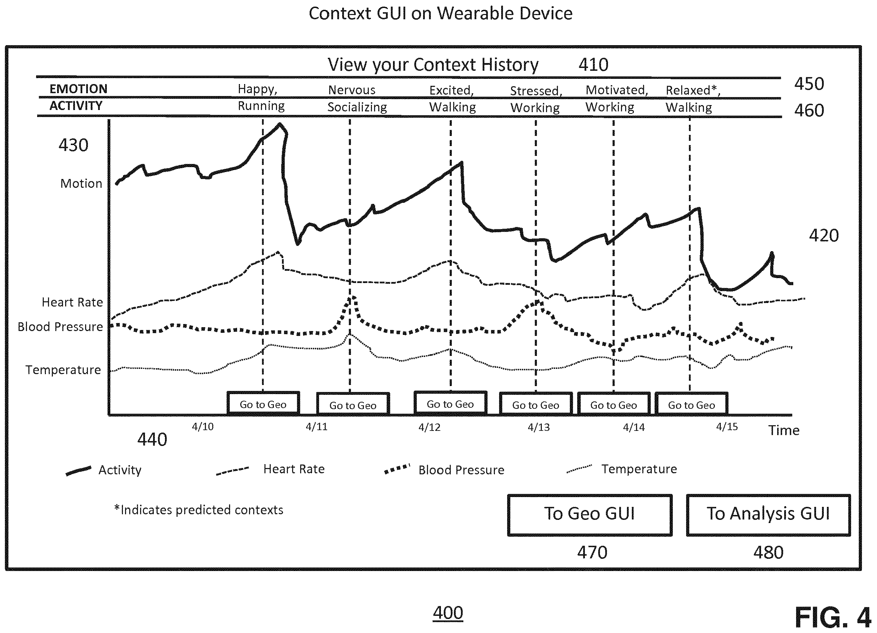

FIG. 4 illustrates an exemplary context GUI 400 that may be rendered and displayed on a display of a wearable device. In one embodiment, context GUI 400 may include a header 410 that identifies GUI 400 as the context GUI (*e.g.,* a title "Context GUI" or a statement such as "View your context history"). Context GUI 400 may include a graphical representation of context data 420, such as a graph. In such embodiments, graph 420 of context GUI 400 may include a vertical axis 430 displaying types of sensor data (*e.g.,* motion sensor data, heart rate sensor data, blood pressure sensor data, and temperature sensor data). The graph of context GUI 400 may include a horizontal axis 440 displaying various times (*e.g.,* April 10^{th}, April 11^{th}, April 12^{th}, April 13^{th}, April 14^{th}, and April 15^{th}). A top of graph 420 may further display, following horizontal axis 440, various types of emotion 450 (*e.g.,* happy, nervous, excited, stressed, motivated, relaxed, etc.) and activities 460 (*e.g.,* running, socializing, walking, working, etc.). Graph 420 may display a point in time that vertically bisects graph 420 and guides the user in viewing specific time points at which sensor data was detected while an emotion 450, activity 460, or other influence was recorded (*e.g.,* inputted by the user) or predicted (*e.g.,* using context learn mode).

Referring from left to right as shown in the example of FIG. 4, emotion 450 labeled "happy" and activity 460 labeled "running" were recorded between April 10^{th} and April 11^{th}. The sensor data is displayed as a peak in the motion data as well as an increase in heart rate and body temperature data. At the bottom of graph 420 following the line beneath the labels "happy" and "running" is a selectable element 470 labeled "To Geo GUI" that, when selected by the user, may identify the geolocation associated with the context (*i.e.* "happy" and "running") by executing a geolocation-oriented GUI.

The second column displays an alternate context in which the user experienced an emotion 450 labeled "nervous" while participating in an activity 460 labeled "socializing." Graph 420 displays that, during combination of the foregoing contextual data (*i.e.,* "nervous" and "socializing"), the motion sensor data detected by the wearable device dropped significantly compared to when the context was "happy" and "running." Graph 420 further displays that the heart rate sensor data decreased and that the data detected by the blood pressure and temperature sensors increased. Using context learn mode, the wearable device may interpret the significant increase in blood pressure to define a prediction rule (*e.g.,* when the user is nervous, the user's blood pressure will increase). The wearable device may further interpret the data to define a prediction rule whereby an increase in temperature data indicates that the user's body temperature will increase in the context of socializing while feeling nervous. When selectable element 470 is selected by the user, the wearable device may identify the geolocation at which the context (*i.e.* "excited" and "walking") occurred.

The third column displays a further example of possible contexts recorded and predicted by the wearable device. The column displays an emotion 450 labeled "excited" and an activity 460 labeled "walking." Graph 420 indicates that, in the context of being "excited" while "walking," the user's motion data increased compared to the context in which the user was socializing. Graph 420 further indicates that the user's motion data did not increase as much as when the user context was "running." Graph 420 further displays that, in the exemplary set of data shown in FIG. 4, the user's heart rate has increased compared to the context in which the user was socializing. Graph 420 further indicates that the user's blood pressure remained steady. At the bottom of the graph following the line beneath the labels "excited" and "walking" is a selectable element labeled "go to geo" that, when selected by the user, navigates the user to the geolocation of the context (*i.e.* "excited" and "walking").

The fourth column displays yet another example of possible contexts recorded and predicted by the wearable device. The column displays an emotion 450 labeled "stressed" that corresponds to an activity 460 labeled "working." Graph 420 indicates that the user's motion data decreased compared to the contexts in which the user was running, walking, or even socializing. Graph 420 further displays that the user's heart rate data has not changed significantly, but that the user's blood pressure has increased significantly in the context of being "stressed" while "working" compared to other contexts (*e.g.,* "happy" and running"). When selectable element 470 is selected by the user, the wearable device may identify the geolocation at which the context (*i.e.* "stressed" and "working") occurred.

The fifth column displays an exemplary context in which a user may be "motivated" and "working." Graph 420 displays changes in sensor data during that particular context. In the example shown, graph 420 reveals that the user's motion (as determined by detected motion sensor data) is lower than when the user is running or walking. The graph reveals other trends and contextual information as well (*e.g.,* that heart rate is normal, that blood pressure has notable decreased compared to when "working" and "stressed," etc.). When selectable element 470 is selected by the user, the wearable device may identify the geolocation at which the context (*i.e.* "motivated" and "working") occurred.

The sixth and final column displays an exemplary context in which the user is "relaxed" and "walking." In context learn mode, the wearable device may analyze the foregoing data and other relationships between data to determine one more context prediction rules. The wearable device may then, in the future, apply the rules based on wearable device sensor data to predict the context in which the wearable device is being used. The context shown in the final column, for example, is displayed with an asterisk to indicate that the context is a predicted context as opposed to a learned context. By indicating that the context is a predicted context, the wearable device may inform the user that the context was generated by, for example, generating and applying a prediction rule by matching the user's current sensor data to a context previously input by the user when the wearable device was detecting the same or similar sensor data. The graphical representation of the sensor data may include a legend correlating the displayed line styles with different types of sensor data.

In some embodiments, context GUI 400 may include one or more selectable elements 470 and 480 that, when selected by a user of the wearable device, may cause the wearable device to render and display a different GUI (*e.g.,* a geo GUI, an analysis GUI, etc.). Selectable elements 470 and 480 may allow a user to navigate between the various available GUIs. The graphical representation shown in FIG. 4 is exemplary and in no way exhaustive of the many ways in which context data may be displayed to a user. Persons of ordinary skill in the art will readily recognize and appreciate that graph 420 is described as an illustrative example only and that many other methods (*e.g.,* a list format) are possible and within the scope of the present disclosure.

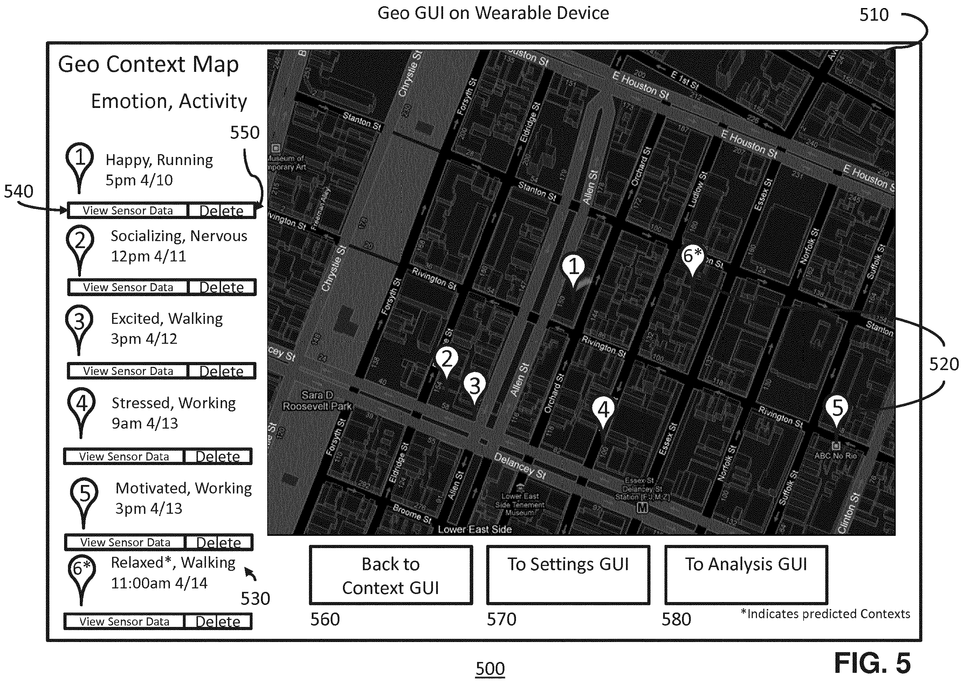

FIG. 5 illustrates an exemplary geo GUI 500 that may be rendered and displayed on a display of a wearable device. In one embodiment, geo GUI 500 may include a map 510 of a geographic area. Map 510 may be a satellite map, a non-satellite map, a two-dimensional map, a three-dimensional map, any combination of the foregoing, or another suitable type of map. Map 510 may include a plurality of points 520 each corresponding to a context 530. For purposes of illustration, the example map 510 shown in FIG. 5 displays points 520 that each correspond to a different context 530 identified in FIG. 4 (*e.g.* "happy, running;" "socializing, nervous;" "excited, walking;" "stressed, working;" "motivated, working;" and "relaxed, walking.). Point 1, for instance, identifies the geolocation (*i.e.,* the corner of Rivington St. and Orchard St.) and time (5:00 PM on April 10) at which the user experienced context 530 labeled "happy, running" and the wearable device detected corresponding sensor data. Map 510 may include additional points 520 corresponding to other contexts 530 (*e.g.,* the wearable device collected certain sensor data while the user was "nervous" and "socializing" at the corner of Delancey St. and Eldridge St at 12:00 PM on April 11). One or more of the points 520 may identify a predicted context 530. As shown in FIG. 5, for instance, point 6 identifies a predicted context 530 of "relaxed" and "walking" for sensor data recorded at 11:00 AM on April 14 at the intersection of Essex St. and Ludlow St. Each point 520 may include a selectable element 540 that, when selected by a user of the wearable device, may cause the wearable device to display sensor data associated with the context 530 by way of a GUI (*e.g.,* "View Sensor Data" button 540). Geo GUI 500 may further include a selectable element 550 associated with each context that, when selected by the user, may cause the wearable device to delete the stored context data (*e.g.,* "Delete" button 550). In some embodiments, geo GUI 500 may also include one or more selectable elements 560, 570, and 580 that, when selected by a user of the wearable device, may cause the wearable device to render and display a different GUI (*e.g.,* a context GUI, a settings GUI, an analysis GUI, etc.). Selectable elements 560, 570, and 580 may allow a user to navigate between the various available GUIs.

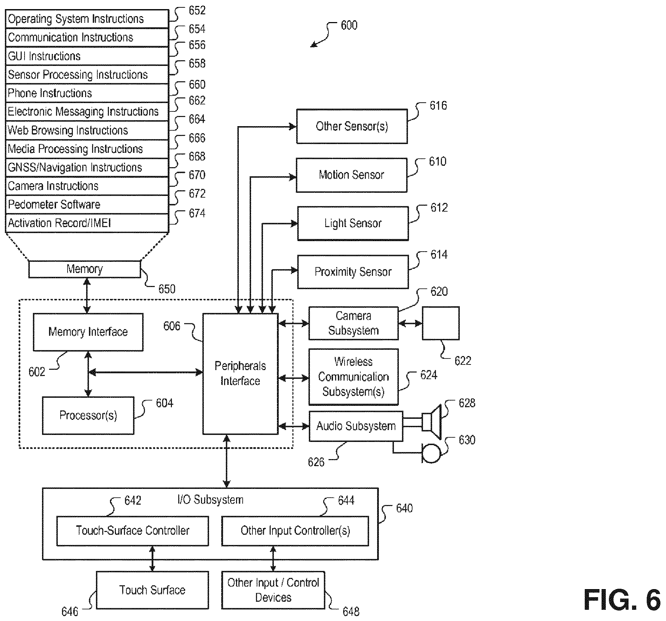

FIG. 6 illustrates an exemplary computing device architecture 600 that may be utilized to implement the various features and processes described herein. Computing device architecture 600 could, for example, be implemented in wearable device 110. Architecture 600 as illustrated in FIG. 6 may include memory interface 602, processors 604, and peripheral interface 606. Memory interface 602, processors 604 and peripherals interface 606 may be separate components or may be integrated as a part of one or more integrated circuits. The various components may be coupled by one or more communication buses or signal lines.

Processors 604, as illustrated in FIG. 6, is meant to be inclusive of data processors, image processors, central processing unit, or any variety of multi-core processing devices. Any variety of sensors, external devices, and external subsystems can be coupled to peripherals interface 606 to facilitate any number of functionalities within the architecture 600 of the exemplar mobile device. For example, motion sensor 610, light sensor 612, and proximity sensor 614 can be coupled to peripherals interface 606 to facilitate orientation, lighting, and proximity functions of the mobile device. For example, light sensor 612 could be utilized to facilitate adjusting the brightness of touch surface 646. Motion sensor 610, which could be exemplified in the context of an accelerometer or gyroscope, could be utilized to detect movement and orientation of the mobile device. Display objects or media could then be presented according to a detected orientation (e.g., portrait or landscape).

Other sensors could be coupled to peripherals interface 606, such as a temperature sensor, a biometric sensor, or other sensing device to facilitate corresponding functionalities. Location processor 615 (*e.g.,* a global positioning transceiver) can be coupled to peripherals interface 606 to allow for generation of geo-location data thereby facilitating geo-positioning. An electronic magnetometer 616 such as an integrated circuit chip could in turn be connected to peripherals interface 606 to provide data related to the direction of true magnetic North whereby the mobile device could enjoy compass or directional functionality. Camera subsystem 620 and an optical sensor 622 such as a charged coupled device (CCD) or a complementary metal-oxide semiconductor (CMOS) optical sensor can facilitate camera functions such as recording photographs and video clips.

Communication functionality can be facilitated through one or more communication subsystems 624, which may include one or more wireless communication subsystems. Wireless communication subsystems 624 can include 802.x or Bluetooth transceivers as well as optical transceivers such as infrared. Wired communication system can include a port device such as a Universal Serial Bus (USB) port or some other wired port connection that can be used to establish a wired coupling to other computing devices such as network access devices, personal computers, printers, displays, or other processing devices capable of receiving or transmitting data. The specific design and implementation of communication subsystem 624 may depend on the communication network or medium over which the device is intended to operate. For example, a device may include wireless communication subsystem designed to operate over a global system for mobile communications (GSM) network, a GPRS network, an enhanced data GSM environment (EDGE) network, 802.x communication networks, code division multiple access (CDMA) networks, or Bluetooth networks. Communication subsystem 624 may include hosting protocols such that the device may be configured as a base station for other wireless devices. Communication subsystems can also allow the device to synchronize with a host device using one or more protocols such as TCP/IP, HTTP, or UDP.

Audio subsystem 626 can be coupled to a speaker 628 and one or more microphones 630 to facilitate voice-enabled functions. These functions might include voice recognition, voice replication, or digital recording. Audio subsystem 626 in conjunction may also encompass traditional telephony functions.

I/O subsystem 640 may include touch controller 642 or other input controller(s) 644. Touch controller 642 can be coupled to a touch surface 646. Touch surface 646 and touch controller 642 may detect contact and movement or break thereof using any of a number of touch sensitivity technologies, including but not limited to capacitive, resistive, infrared, or surface acoustic wave technologies. Other proximity sensor arrays or elements for determining one or more points of contact with touch surface 646 may likewise be utilized. In one implementation, touch surface 646 can display virtual or soft buttons and a virtual keyboard, which can be used as an input/output device by the user.

Other input controllers 644 can be coupled to other input/control devices 648 such as one or more buttons, rocker switches, thumb-wheels, infrared ports, USB ports, or a pointer device such as a stylus. The one or more buttons (not shown) can include an up/down button for volume control of speaker 628 or microphone 630. In some implementations, device 600 can include the functionality of an audio or video playback or recording device and may include a pin connector for tethering to other devices.

Memory interface 602 can be coupled to memory 650. Memory 650 can include high-speed random access memory or non-volatile memory such as magnetic disk storage devices, optical storage devices, or flash memory. Memory 650 can store operating system 652, such as Darwin, RTXC, LINUX, UNIX, OS X, ANDROID, WINDOWS, or an embedded operating system such as VxWorks. Operating system 652 may include instructions for handling basic system services and for performing hardware dependent tasks. In some implementations, operating system 652 can include a kernel.

Memory 650 may also store communication instructions 654 to facilitate communicating with other mobile computing devices or servers. Communication instructions 654 can also be used to select an operational mode or communication medium for use by the device based on a geographic location, which could be obtained by the GPS/Navigation instructions 668. Memory 650 may include graphical user interface instructions 656 to facilitate graphic user interface processing such as the generation of an interface; sensor processing instructions 658 to facilitate sensor-related processing and functions; phone instructions 660 to facilitate phone-related processes and functions; electronic messaging instructions 662 to facilitate electronic-messaging related processes and functions; web browsing instructions 664 to facilitate web browsing-related processes and functions; media processing instructions 666 to facilitate media processing-related processes and functions; GPS/Navigation instructions 668 to facilitate GPS and navigation-related processes, camera instructions 670 to facilitate camera-related processes and functions; and instructions 672 for any other application that may be operating on or in conjunction with the mobile computing device. Memory 650 may also store other software instructions for facilitating other processes, features and applications, such as applications related to navigation, social networking, location-based services or map displays.

Each of the above identified instructions and applications can correspond to a set of instructions for performing one or more functions described above. These instructions need not be implemented as separate software programs, procedures, or modules. Memory 650 can include additional or fewer instructions. Furthermore, various functions of the mobile device may be implemented in hardware or in software, including in one or more signal processing or application specific integrated circuits.

Certain features may be implemented in a computer system that includes a back-end component, such as a data server, that includes a middleware component, such as an application server or an Internet server, or that includes a front-end component, such as a client computer having a graphical user interface or an Internet browser, or any combination of the foregoing. The components of the system can be connected by any form or medium of digital data communication such as a communication network. Some examples of communication networks include LAN, WAN and the computers and networks forming the Internet. The computer system may include clients and servers. A client and server are generally remote from each other and typically interact through a network. The relationship of client and server arises by virtue of computer programs running on the respective computers and having a client-server relationship to each other.

One or more features or steps of the disclosed embodiments may be implemented using an API that can define on or more parameters that are passed between a calling application and other software code such as an operating system, library routine, function that provides a service, that provides data, or that performs an operation or a computation. The API can be implemented as one or more calls in program code that send or receive one or more parameters through a parameter list or other structure based on a call convention defined in an API specification document. A parameter can be a constant, a key, a data structure, an object, an object class, a variable, a data type, a pointer, an array, a list, or another call. API calls and parameters can be implemented in any programming language. The programming language can define the vocabulary and calling convention that a programmer will employ to access functions supporting the API. In some implementations, an API call can report to an application the capabilities of a device running the application, such as input capability, output capability, processing capability, power capability, and communications capability.

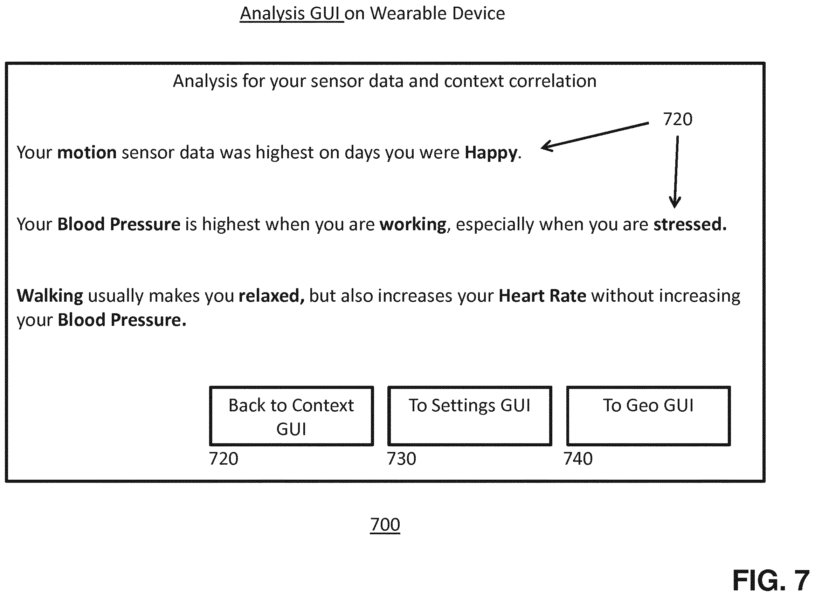

FIG. 7 illustrates an exemplary analysis GUI 700 that may be rendered and displayed on a display of a wearable device. Analysis GUI 700 may display analysis information to the user of the wearable device. Analysis GUI 700 may, for instance, display correlation data concerning how sensor data relates to the context in which it was collected. In one exemplary scenario, the analysis GUI 700 may display correlation data as a textual statement 710 (*e.g.,* "Your motion sensor data was highest on days when you were happy.") In doing so, analysis GUI 700 may report how the sensor data (*e.g.,* motion sensor data) correlates to an activity, emotion, or other influence (*e.g.,* happiness). Analysis GUI 700 may display a text statement 710 such as "Your blood pressure is highest when you are working, especially when you are stressed." Analysis GUI 700 may display certain words in a distinct typeface to emphasize the words. For instance, in the foregoing statement, analysis GUI 700 may emphasize the type of sensor data at issue (*e.g.,* blood pressure), the activity that constitutes the contextual information (*e.g.,* working), and the emotion that constitutes additional contextual information (*e.g.,* being stressed). Analysis GUI 700 may further report correlations by displaying statements such as "Walking usually makes you relaxed, but also increases your heart rate without increasing your blood pressure." In the foregoing example, analysis GUI 700 may emphasize the activity constituting the contextual information (*e.g.,* walking), the feeling or emotion constituting additional contextual information (*e.g.,* feeling relaxed), and the two types of sensor data being monitored (*e.g.,* heart rate and blood pressure data). In some embodiments, analysis GUI 700 may include one or more selectable elements 720, 730, and 740 that, when selected by a user of the wearable device, may cause the wearable device to render and display a different GUI (*e.g.,* a context GUI, a settings GUI, a geo GUI, etc.). Selectable elements 720, 730, and 740 may allow a user to navigate between the various available GUIs.

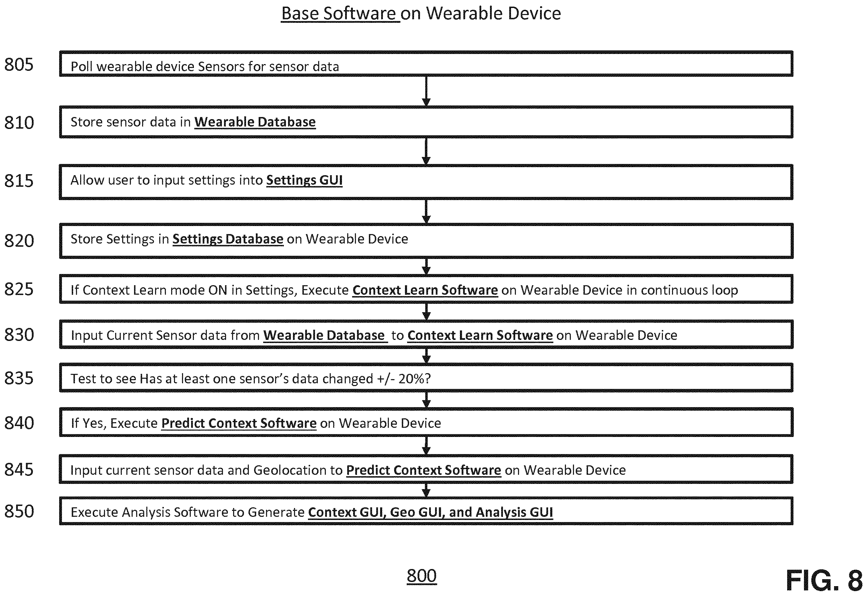

FIG. 8 illustrates an exemplary operational process performed by a base software module 800 stored in memory and executed by a processor of a wearable device. Upon execution by a processor of the wearable device (*e.g.,* wearable device 100 of FIG. 1), base software module may poll one or more wearable device sensors for sensor data at step 805. At step 810, the polled sensor data may be stored in a wearable database stored in memory of the wearable device. Input settings may, at step 815, be received from a user of the wearable device by way of a settings GUI. At step 820, the received settings may be stored in a settings database stored in memory of the wearable device. The context learn mode may have an "on" setting and an "off' setting, each of which may be specified in the settings received from the user. At step 825, when context learn mode is set to "on" in the received settings (*i.e.,* activated or enabled), the base software module may cause the processor to execute a context learn software module in a continuous loop. At step 830, the base software module may then pass sensor data stored in the wearable database to the context learn software module. The base software module may then, at step 835, determine whether sensor data associated with at least one sensor has changed. In some embodiments, the base software module may determine whether sensor data has changed at all, while in other embodiments the base software module may determine whether sensor data has changed enough to satisfy a predetermined threshold (*e.g.,* +/- 20%) fall into a predetermined range (*e.g.,* heart rate between 100 and 110 beats per minute). At step 840, when the base software module determines that sensor data has changed (to any degree or enough to satisfy a predetermined threshold or range, depending on the embodiment), the base software module may cause the processor of the wearable device to execute a predict context software module stored in memory of the wearable device. At step 845, the base software module may then pass current sensor data and geolocation data to the predict context software module. The base software module may then, at step 850, execute an analysis software module and generate a plurality of GUIs (*e.g.,* a context GUI, a geo GUI, or an analysis GUI) on display of the wearable device.

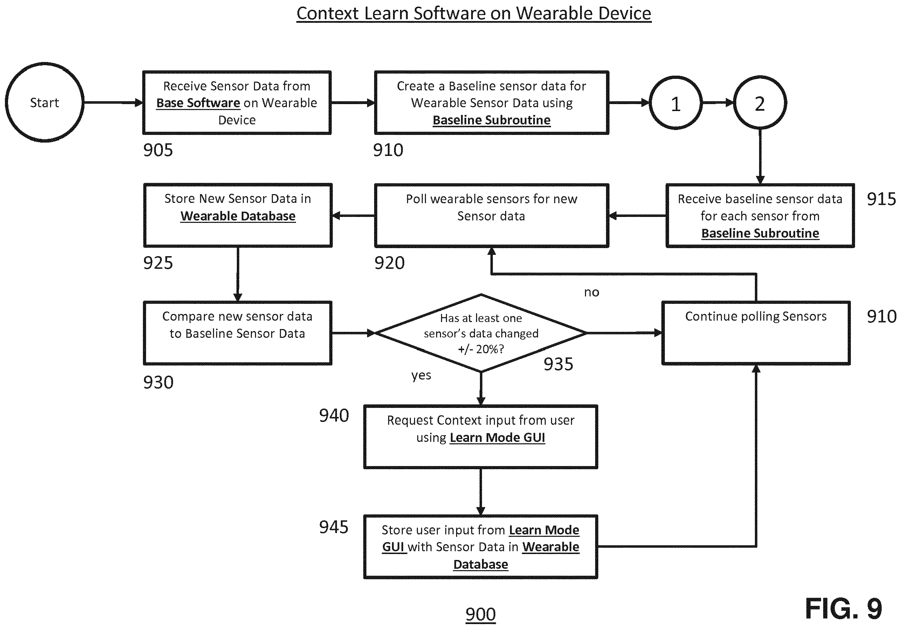

FIG. 9 illustrates an exemplary operational process 900 performed by a context learn software module stored in memory and executed by a processor of a wearable device. Upon execution by a processor of the wearable device (*e.g.,* wearable device 100 of FIG. 1), the context learn software module may receive sensor data from the base software module at step 905. The context learn software module may, by way of executing a baseline subroutine, create a baseline sensor data for wearable sensor data at step 910.

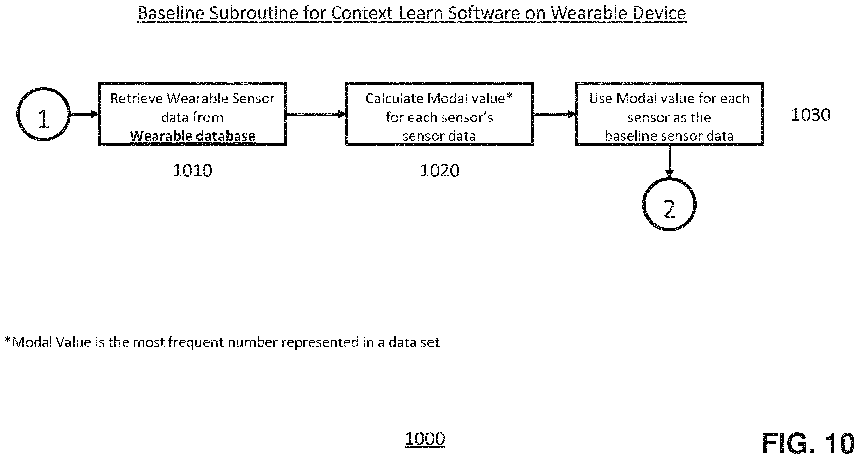

FIG. 10 illustrates an exemplary baseline subroutine 1000 performed by a context learn software module stored in memory and executed by a processor of a wearable device. Upon execution by a processor of the wearable device (*e.g.,* wearable device 100 of FIG. 1), the baseline subroutine of the context learn software module may, at step 1010, retrieve sensor data (*e.g.,* raw sensor data or parameters extracted therefrom) from the wearable database stored in memory of the wearable device. At step 1020, the baseline subroutine may then calculate a modal value for each sensor's data. A modal value is the most frequent number represented in a data set (*i.e.,* the statistical mode). The baseline subroutine may then, at step 1030, establish the modal value for each sensor as the baseline sensor data for that particular sensor. It will be apparent that various alternative methods for establishing a baseline value will be apparent. For example, the mean or median of the data set may be selected as a baseline. In other embodiments, a baseline value may be represented by a value other than a single number such as a range of values. For example, the baseline value may be represented by a range bounded by the 25^{th} and 75^{th} percentile values in the data set. In various embodiments, the data set from which the baseline in derived may be the data set of all readings from the sensor, samples from throughout the lifetime of the sensor, or samples within a recent time window (*e.g.,* the last 10 minutes or the last 2 hours). In some embodiments, the method used to calculate the baseline may vary from sensor to sensor.

Referring back to FIG. 9, the context learn software module may, at step 915, receive the baseline sensor data for each sensor from the baseline subroutine. At step 920, the context learn software module may then poll one or more of the wearable sensors for new sensor data. Any new sensor data received as a result of the polling operation may, at step 925, be stored in the wearable database stored in memory of the wearable device. At step 930, the context learn software module may then compare the new sensor data to the baseline sensor data. As illustrated at step 935, the context learn software module may determine whether at least one sensor's data has changed. In some embodiments, the context learn software module may determine whether sensor data has changed at all from the baseline, while in other embodiments the context learn software module may determine whether sensor data has changed enough to satisfy a predetermined threshold (*e.g.,* +/- 20% of the calculated baseline) or to fall into a predetermined range (*e.g.,* heart rate between 100 and 110 beats per minute). In some embodiments, a single sensor value may be sufficient to determine that the sensor data has changed while, in other embodiments, the new sensor data may include values from multiple (*e.g.,* multiple consecutive) pollings of the sensors or extractions of parameters. In some such embodiments, multiple contemporary sensor values may need to be judged to have "changed" before the sensor data as a whole is deemed "changed" for purposes of identifying a new context for which user input will be requested. For example, in some embodiments, a threshold of values (*e.g.,* 4 of the last 5 or 75% of recently polled values) may be required to have changed before the new sensor data as a whole is treated as changed. In other embodiments, an average or modal value of the new sensor data may be used for steps 930-935.

At step 940, when the context learn software module determines that sensor data has changed (to any degree or enough to satisfy a predetermined threshold or range, depending on the embodiment), the context learn software module may request context input from the user by way of a learn mode GUI. The context learn software module may cause the processor of the wearable device to execute a learn mode GUI module that, when executed, may render and display the learn mode GUI on a display of the wearable device. In some alternative embodiments, such as those embodiments wherein the wearable device does not include a user interface for receiving such input, the wearable device may communicate with another device (*e.g.,* an app on a user's mobile phone, tablet, or pc) to obtain the context input.

At step 945, the context learn software module may store any input received by way of the learn mode GUI with sensor data stored in the wearable database. The context learn software module may then continue polling the one or more sensors for further sensor data as described in the context of step 910. When the context learn software module determines that sensor data has not changed (either to any degree or not enough to satisfy a predetermined threshold or range, depending on the embodiment), the context learn software module may continue polling the one or more sensors for further sensor data. The processor may then proceed in a continuous monitoring loop in which the one or more sensors are polled for new sensor data, new sensor data is received, stored, and compared to baseline sensor data, and the data is evaluated for changes. When the context learn software module is executed in a loop as shown in FIG. 9, context learn mode may passively run while the user operates the wearable device. The wearable device may continuously learn about the context in which it is used and may ultimately predict contexts based on previously acquired data.

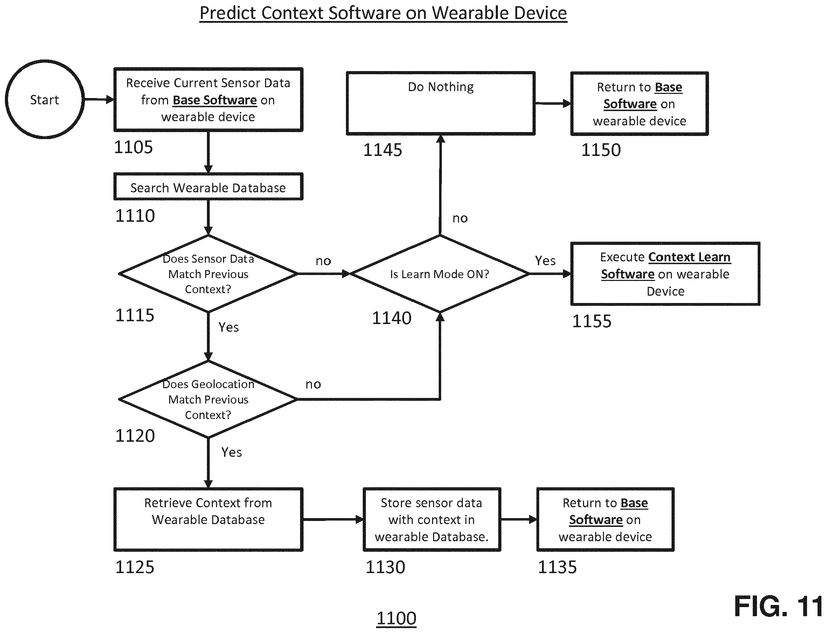

FIG. 11 illustrates an exemplary operational process 1100 performed by a predict context software module stored in memory and executed by a processor of a wearable device. Upon execution by a processor of the wearable device (*e.g.,* wearable device 100 of FIG. 1) at step 1105, the predict context software module may receive real-time sensor data from the base software module. At step 1110, the predict context software module may search the wearable database stored in memory of the wearable device. The predict context software module may, at step 1115, determine whether received sensor data matches a previous context. At step 1120, when the predict context software module determines that received sensor data matches a previous context, the predict context software module may determine whether geolocation data associated with the received sensor data matches a previous context. When the geolocation data associated with the received sensor data matches a previous context, the predict context software module may, at step 1125, retrieve context information from the wearable database. At step 1130, the predict context software module may then store the received sensor data with the context information in the wearable database. At step 1135, the predict context software module may instruct the processor to execute the base software module.

When the predict context software module determines that received sensor data does not match a previous context, as shown at step 1140, the predict context software module may determine whether context learn mode is "on" (*i.e.,* activated or enabled) as dictated by the settings stored in a settings database. At step 1145, when the predict context software module determines that context learn mode is not "on" (*i.e.,* is "off'), the predict context software module may take no further action other than causing the processor to return to the operations of the executing base software module at step 1150. At step 1155, when the predict context software module determines that context learn mode is "on," the predict context software module may cause the processor of the wearable device to execute the context learn software module stored in memory of the wearable device.

In embodiments in which the predict context software module determines, at step 1120, whether geolocation data associated with the received sensor data matches a previous context, the predict context software module may return to step 1140 and determine whether learn mode is "on" when the geolocation data associated with the received sensor data does not match a previous context. As noted above, when the predict context software module determines that context learn mode is not "on" (*i*.*e.,* is "off'), the predict context software module may, at step 1145, take no further action other than causing the processor to return to the operations of the executing base software module. When the predict context software module determines that context learn mode is "on," the predict context software module may, at step 1155, cause the processor of the wearable device to execute the context learn software module stored in memory of the wearable device.

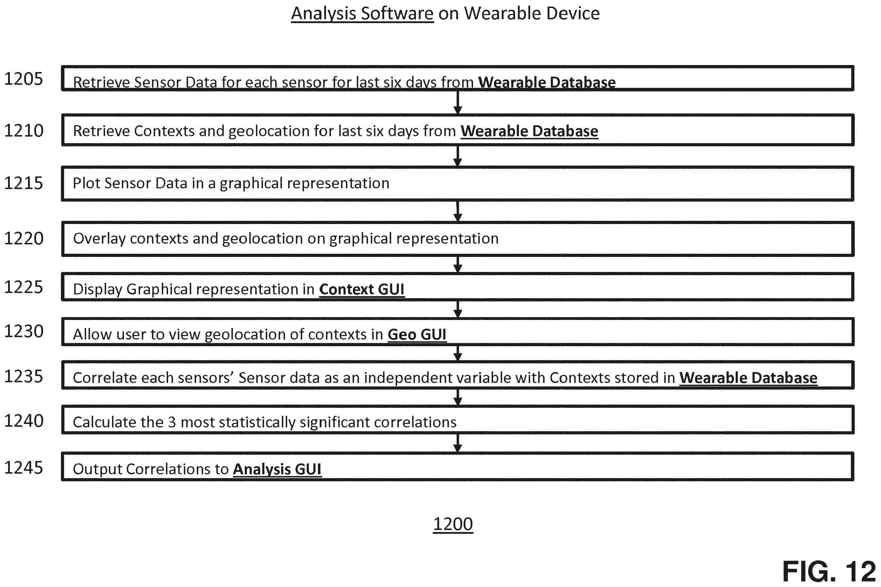

FIG. 12 illustrates an exemplary operational process 1200 performed by an analysis software module stored in memory and executed by a processor of a wearable device. Upon execution by a processor of the wearable device (*e.g.,* wearable device 100 of FIG. 1) at step 1205, the analysis software module may retrieve from the wearable database, for one or more of the sensors disposed in the wearable device, sensor data obtained over a previous timeframe (*e.g.,* over the last six days). At step 1210, the analysis software module may then retrieve from the wearable database, for the one or more sensors, context and geolocation data obtained over the previous timeframe (*e.g.,* over the last six days). The analysis software module may then, at step 1215, render the sensor data, context data, and geolocation data as a graphical representation. The analysis software module may, for instance, plot the data as a graph. At step 1220, the analysis software module may overlay context and geolocation data within the graphical representation and display the graphical representation by way of a context GUI. At step 1225, the analysis software module may cause the processor of the wearable device to render and display the context GUI on a display of the wearable device. The analysis software module may, at step 1230, further display geolocation data corresponding to various portions of context data by way of a geo GUI. The analysis software module may cause the processor of the wearable device to render and display the geo GUI on a display of the wearable device. At step 1235, the analysis software module may correlate each sensor's data as an independent variable with one or more contexts represented by context data stored in the wearable database. The analysis software module may then, at step 1240, calculate one or more statistics, such as the three most statistically significant correlations. At step 1245, the analysis software module may output the calculated correlations to an analysis GUI, which may display the correlations to the user. The analysis software module may cause the processor of the wearable device to render and display the analysis GUI on a display of the wearable device.

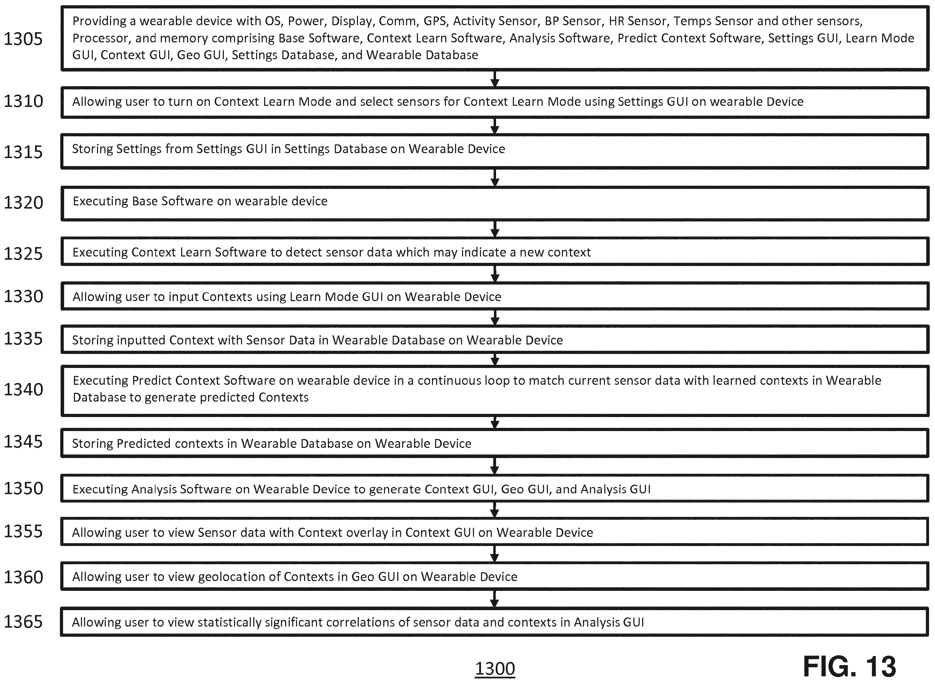

FIG. 13 illustrates an exemplary method 1300 for predicting a context in which a wearable health device is used by a user based on contextual information previously supplied by the user. Method 1300 may include, at step 1305, providing a wearable device like that described in the context of FIG. 1. The wearable device may include a plurality of components connected by one or more buses, including a processor, memory, a power supply, a display, one or more sensors (*e.g.,* an activity sensor, a blood pressure sensor, a heart rate sensor, a temperature sensor, or other sensor), and a wired or wireless communications module (*e.g.,* a USB port module, a FireWire port module, a Lightning port module, a Thunderbolt port module, a Wi-Fi connection module, a 3G/4G/LTE cellular connected module, a Bluetooth connection module, a lower powered Bluetooth connection module, a near field communication module, etc.). The components may further include a GPS module. The wearable device may be operable to store in memory, and the processor of the wearable device may be operable to execute, a wearable device OS and a plurality of executable software modules. The executable software modules may include a base software module, a context learn software module, an analysis software module, a predict context software module, and one or more GUI software modules that, when executed, render and display one or more GUIs on a display of the wearable device. The one or more GUI software modules may include a settings GUI module, a learn mode GUI module, a context GUI module, a geo GUI module, and an analysis GUI module. The wearable device may further be operable to store one or more databases in memory (*e.g.,* a settings database, a wearable database, etc.).

Method 1300 may include, at step 1310, allowing a user to turn on context learn mode and selecting one or more sensors for context learn mode. Allowing the user to do so may include receiving one or more setting selections by way of a settings GUI rendered and displayed on a display of the wearable device. At step 1315, method 1300 may include storing settings received by way of the settings GUI in a settings database stored in memory of the wearable device. Method 1300 may further include executing a base software module stored in memory of the wearable device at step 1320. Method 1300 may also include, at step 1325, executing a context learn software module stored in memory of the wearable device. Upon being executed by a processor of the wearable device, the context learn software module may detect sensor data that may indicate a new context associated with received sensor data.

Method 1300 may further include allowing the user to input context information to be associated with sensor data at step 1330. The context information and sensor data may be received by the wearable device and, at step 1335, may be stored in memory (*e.g.,* in a wearable database). Allowing the user to input context information may include receiving context information from the user by way of a learn mode GUI rendered and displayed on a display of the wearable device. The learn mode GUI may be rendered and displayed during execution of a learn mode GUI module. The method may include storing received context information with sensor data in the wearable database.

At step 1340, method 1300 may further include executing a predict context software module stored in memory of the wearable device. Executing the predict context software module may include executing the module in a continuous loop so as to match received sensor data with learned contexts stored in the wearable database and generate predicted contexts. At step 1345, method 1300 may include storing predicted contexts in memory (*e.g.,* in the wearable database).

Method 1300 may also include executing an analysis software module stored in memory of the wearable device at step 1350. Execution of the analysis software module may result in the rendering and display of one or more GUIs, such as a context GUI, a geo GUI, and an analysis GUI. Method 1300 may include, at step 1355, allowing a user to view various data by way of displaying the data through various the GUIs displayed on a display of the wearable device. Method 1300 may include displaying sensor data with overlaid context data in a context GUI. At step 1360, method 1300 may also include displaying geolocation data associated with one or more contexts represented by context data by way of a geo GUI displayed on the wearable device. Method 1300 may further include displaying one or more statistical elements at step 1365, such as a statistically significant correlation between sensor data and context. The statistical elements may be displayed by way of an analysis GUI rendered and displayed at a display of the wearable device.

The foregoing method steps have been described in one of many possible ordered sequences for illustrative purposes. Persons of ordinary skill in the art will readily appreciate that certain steps may be omitted or performed in a different order depending on the overall system architecture.

In various embodiments, the wearable device disclosed herein (*e.g.,* wearable device 100 of FIG. 1) may detect the presence of other wearable devices (*e.g.,* wearable devices within a given proximity or connected to a common network or through Bluetooth™ connectivity or the like). The wearable device may exchange data with the detected other wearable device to enhance contexts and the context learn mode. As a result, the wearable device may expedite its learning rate by combining data from multiple wearable device users. The wearable device may also automatically detect and evaluate that a user of a first wearable device performed a particular exercise (*e.g.,* running) or experienced a particular emotion or other influence (*e.g.,* feeling motivated) while accompanied by a user of a second wearable device (*e.g.,* a friend of the user of the first wearable device). The wearable device may interpret the presence of a second user as a context in and of itself.

In various embodiments, the wearable device may provide access to learned contexts to a third-party system or network. The wearable device may analyze data and determine when a user is most effective at performing a particular activity (*e.g.,* an exercise). The wearable device may also analyze data and determine when a user experiences the most positive emotions or feelings based on geolocation. The wearable device may analyze sensor data and contexts and display a map of a user's preferred or "happy" places. The displayed information may include times or locations at which sensor data indicates the user was exercising harder, running further or faster, experiencing happiness for longer periods of time, etc. As a result, the device may provide the user with an improved understanding of his or her own emotional and activity context data. In some embodiments, the wearable device may provide functionality by which a user may "pin" or otherwise designate learned contexts or set reminders for contexts to be achieved at specified time intervals (*e.g.,* on a daily basis).

In various embodiments, the wearable device may provide functionality by which a user may enhance context data with additional data (*e.g.,* hydration, caloric intake, injuries, or health condition information). Not only may the user input emotion and activity data, but the user may submit surveys or questionnaires that provide further detail (*e.g.,* how much water the user drank in a given day, etc.).

In one or more embodiments, the wearable device may include a software module that automatically executes when a context is predicted. The software module may compare received data in real time to a user's previous context. For example, where a predicted context is "happy and walking," the wearable device may show the current data and the data received the last time the user was "happy and walking." As a result, the user may compare sensor data for two events in real-time. In an example in which a user is exercising, the user may see the last time he or she was walking or running and have a sort of "ghost" of themselves to which they can compare their current activity. The user may determine whether they are walking faster, farther, or shorter and slower, compared to a previous context.

In various embodiments, the wearable device may include functionality by which learned contexts automatically trigger third-party application functionality (*e.g.,* updating a calendar, note, or journal entry). The functionality, which may be carried out by a software module executed by a processor the wearable device, may permit a user to set up custom triggers that, when triggered by an event at the wearable device, automatically execute an application of a wearable device or a smartphone based on learned and predicted contexts.

The foregoing detailed description has been presented for purposes of illustration and description. It is not intended to be exhaustive or to limit the technology to the precise form disclosed. Many modifications and variations are possible in light of the above teaching. The described embodiments were chosen in order to best explain the principles of the technology and its practical application to enable others skilled in the art to best utilize it in various embodiments and with various modifications as suited to the particular design considerations at issue (e.g., cost, availability, preference, etc.). The scope of the technology should be defined only by the claims appended to this description.

## Claims

1. A wearable device (100) comprising:
a sensor (130, 135, 140, 145, 150) configured to obtain sensor data descriptive of a physiological parameter of a user;
a memory (115) configured to store a plurality of records correlating context data to physiological parameters obtained from sensor data; and
a processor (110) configured to:
establish a baseline value based on a first set of sensor data obtained from the sensor (130, 135, 140, 145, 150) and retrieved from memory (115),
poll the sensor (130, 135, 140, 145, 150) for new sensor data to obtain a second set of sensor data,
compare the second set of sensor data obtained from the sensor (130, 135, 140, 145, 150) to the baseline value to determine whether the second set of sensor data is a change from the baseline value,
request user input from the user descriptive of a current context in response to determining that the second set of sensor data is a change from the baseline value,
create a record based on the second set of sensor data and the current context, and
store the created record in the memory (115) as a member of the plurality of records.

2. The wearable device (100) of claim 1, further comprising a communication interface (155) configured to communicate over a communication network, wherein the communication interface (155) is configured to detect the presence of one or more other wearable devices communicatively coupled to the network.

3. The wearable device (100) of claim 2, wherein the communication interface (155) is further configured to receive additional context data from the one or more other wearable devices communicatively coupled to the network, and wherein the created record includes the additional context data.

4. The wearable device (100) of claim 1, wherein the processor (110) is further configured to:
train a machine-learning model using the plurality of records; and
apply the machine-learning model to a third set of sensor data obtained from the sensor (130, 135, 140, 145, 150) at a later time to estimate a context at the later time.

5. The wearable device (100) of claim 1, wherein establishing the baseline value comprises obtaining a statistical mode figure from the first set of sensor data.

6. A method for training a wearable device (100) to predict a context in which a wearable device (100) is used, the method comprising:
establishing a baseline value based on a first set of sensor data obtained from a sensor (130, 135, 140, 145, 150) configured to obtain sensor data descriptive of a physiological parameter of a user and the first set of sensor data retrieved from a memory (115) of the wearable device;
polling the sensor (130, 135, 140, 145, 150) for new sensor data to obtain a second set of sensor data;
comparing the second set of sensor data obtained from the sensor (130, 135, 140, 145, 150) to the baseline value to determine whether the second set of sensor data is a change from the baseline value;
requesting user input from the user descriptive of a current context in response to determining that the second set of sensor data is a change from the baseline value;
creating a record based on the second set of sensor data and the current context; and
storing the created record in a memory (115) as a member of a plurality of records.

7. The method of claim 6, further comprising detecting the presence of one or more other wearable devices communicatively coupled to the network.

8. The method of claim 7, further comprising receiving additional context data from the one or more other wearable devices communicatively coupled to the network, wherein the created record includes the additional context data.

9. The method of claim 6, wherein establishing the baseline value comprises obtaining a statistical mode figure from the first set of sensor data.

10. The method of claim 6, further comprising
training a machine-learning model using the plurality of records; and
applying the machine-learning model to a third set of sensor data obtained from the sensor (130, 135, 140, 145, 150) at a later time to estimate a context at the later time.

11. A non-transitory computer-readable medium having a computer program stored thereon, the computer program executable by a processor (110) to perform a method for predicting a context in which a wearable health device is used, the method comprising:
instructions for establishing a baseline value based on a first set of sensor data obtained from a sensor (130, 135, 140, 145, 150) configured to obtain sensor data descriptive of a physiological parameter of a user and the first set of sensor data retrieved from a memory (115) of the wearable device;
instructions for polling the sensor (130, 135, 140, 145, 150) for new sensor data to obtain a second set of sensor data;
instructions for comparing the second set of sensor data obtained from the sensor (130, 135, 140, 145, 150) to the baseline value to determine whether the second set of sensor data is a change from the baseline value;
instructions for requesting user input from the user descriptive of a current context in response to determining that the second set of sensor data is a change from the baseline value;
instructions for creating a record based on the second set of sensor data and the current context; and
instructions for storing the created record in a memory (115) as a member of a plurality of records.

12. The non-transitory computer-readable medium of claim 11, further comprising instructions for detecting the presence of one or more other wearable devices communicatively coupled to the network.

13. The non-transitory computer-readable medium of claim 12, further comprising instructions for receiving additional context data from the one or more other wearable devices communicatively coupled to the network, wherein the created record includes the additional context data.

14. The non-transitory computer-readable medium of claim 11, wherein the instructions for establishing the baseline value comprise instructions for obtaining a statistical mode figure from the first set of sensor data.

15. The non-transitory computer-readable medium of claim 11, further comprising
instructions for training a machine-learning model using the plurality of records; and
instructions for applying the machine-learning model to a third set of sensor data obtained from the sensor (130, 135, 140, 145, 150) at a later time to estimate a context at the later time.

## Patentansprüche

1. Tragbares Gerät (100), das aus Folgendem besteht:
einem Sensor (130, 135, 140, 145, 150), der so konfiguriert ist, dass er Sensordaten erhält, die einen physiologischen Parameter eines Benutzers beschreiben;
einem Speicher (115), der so konfiguriert ist, dass er eine Vielzahl von Datensätzen speichert, die Kontextdaten mit physiologischen Parametern korrelieren, die aus Sensordaten erhalten werden; und
einem Prozessor (110), der dazu konfiguriert ist:
einen Basislinienwert auf Grundlage eines ersten Satzes von Sensordaten festzulegen, die vom Sensor (130, 135, 140, 145, 150) empfangen und aus dem Speicher abgerufen wurden (115),
den Sensor (130, 135, 140, 145, 150) nach neuen Sensordaten abzufragen, um einen zweiten Satz von Sensordaten zu erhalten,
den zweiten Satz von Sensordaten, die vom Sensor (130, 135, 140, 145, 150) empfangen wurden, mit dem Basislinienwert vergleichen, um festzustellen, ob der zweite Satz von Sensordaten eine Änderung gegenüber dem Basislinienwert darstellt,
Benutzereingaben vom Benutzer anzufordern, die einen aktuellen Kontext beschreiben, als Reaktion auf die Feststellung, dass der zweite Satz von Sensordaten eine Änderung vom Basislinienwert aufweist,
einen Datensatz auf Grundlage des zweiten Satzes von Sensordaten und des aktuellen Kontexts zu erstellen, und
den erstellten Datensatz im Speicher (115) als ein Mitglied der Mehrzahl von Datensätzen zu speichern.

2. Tragbares Gerät (100) nach Anspruch 1, die ferner eine Kommunikationsschnittstelle (155) umfasst, die so konfiguriert ist, dass sie über ein Kommunikationsnetz kommuniziert, wobei die Kommunikationsschnittstelle (155) so konfiguriert ist, dass sie das Vorhandensein einer oder mehrerer anderer tragbarer Geräte erfasst, die kommunikativ mit dem Netz gekoppelt sind.

3. Tragbares Gerät (100) nach Anspruch 2, wobei die Kommunikationsschnittstelle (155) ferner so konfiguriert ist, dass sie zusätzliche Kontextdaten von dem einen oder mehreren anderen tragbaren Geräten empfängt, die kommunikativ mit dem Netzwerk gekoppelt sind, und wobei der erstellte Datensatz die zusätzlichen Kontextdaten enthält.

4. Tragbares Gerät (100) nach Anspruch 1, wobei der Prozessor (110) ferner dazu konfiguriert ist:
ein maschinelles Lernmodell unter Verwendung der Vielzahl von Datensätzen zu trainieren; und
das maschinelle Lernmodell auf einen dritten Satz von Sensordaten anzuwenden, die vom Sensor (130, 135, 140, 145, 150) zu einem späteren Zeitpunkt empfangen werden, um zu einem späteren Zeitpunkt einen Kontext abzuschätzen.

5. Tragbares Gerät (100) nach Anspruch 1, wobei die Bestimmung des Basislinienwertes das Empfangen einer statistischen Moduszahl aus dem ersten Satz von Sensordaten umfasst.

6. Verfahren zum Trainieren eines tragbaren Geräts (100), um einen Kontext vorherzusagen, in dem ein tragbares Gerät (100) verwendet wird, wobei das Verfahren Folgendes umfasst:
Festlegung eines Basislinienwertes auf Grundlage eines ersten Satzes von Sensordaten, die von einem Sensor (130, 135, 140, 145, 150) empfangen werden, der so konfiguriert ist, dass er Sensordaten, die einen physiologischen Parameter eines Benutzers beschreiben, und den ersten Satz von Sensordaten, der aus einem Speicher (115) des tragbaren Geräts abgerufen wird, erhält;
Abfrage des Sensors (130, 135, 140, 145, 150) nach neuen Sensordaten, um einen zweiten Satz von Sensordaten zu empfangen;
Vergleich des zweiten Satzes von Sensordaten, die vom Sensor (130, 135, 140, 145, 150) empfangen wurden, mit dem Basislinienwert, um festzustellen, ob der zweite Satz von Sensordaten eine Änderung gegenüber dem Basislinienwert darstellt;
Anforderung von Benutzereingaben vom Benutzer, die einen aktuellen Kontext beschreiben, als Reaktion auf die Feststellung, dass der zweite Satz von Sensordaten eine Änderung gegenüber dem Ausgangswert darstellt;
Erstellen eines Datensatzes auf Grundlage des zweiten Satzes von Sensordaten und des aktuellen Kontext; und
Speichern des erzeugten Datensatzes in einem Speicher (115) als ein Mitglied einer Vielzahl von Datensätzen.

7. Verfahren nach Anspruch 6, das ferner die Feststellung des Vorhandenseins eines oder mehrerer anderer tragbarer Geräte umfasst, die kommunikativ an das Netzwerk gekoppelt sind.

8. Verfahren nach Anspruch 7, das ferner das Empfangen zusätzlicher Kontextdaten von dem einen oder mehreren anderen tragbaren Geräten umfasst, die kommunikativ mit dem Netzwerk gekoppelt sind, wobei der erzeugte Datensatz die zusätzlichen Kontextdaten enthält.

9. Verfahren nach Anspruch 6, wobei die Bestimmung des Basislinienwertes den Empfang einer statistischen Moduszahl aus dem ersten Satz von Sensordaten umfasst.

10. Verfahren nach Anspruch 6, das ferner das Trainieren eines maschinell lernenden Modells unter Verwendung der Vielzahl von Datensätzen umfasst; und Anwenden des maschinell lernenden Modells auf einen dritten Satz von erhaltenen Sensordaten von dem Sensor (130, 135, 140, 145, 150) zu einem späteren Zeitpunkt, um zu einem späteren Zeitpunkt einen Kontext abzuschätzen.

11. Nicht-vorübergehendes computerlesbares Medium mit einem darauf gespeicherten Computerprogramm, wobei das Computerprogramm durch einen Prozessor (110) ausführbar ist, um ein Verfahren zum Vorhersagen eines Kontexts auszuführen, in dem ein tragbares Gesundheitsgerät verwendet wird, wobei das Verfahren Folgendes umfasst:
Anweisungen zum Festlegen eines Basislinienwertes auf Grundlage eines ersten Satzes von Sensordaten, die von einem Sensor (130, 135, 140, 145, 150) erhalten werden, der dazu konfiguriert ist, Sensordaten zu erhalten, die einen physiologischen Parameter eines Benutzers beschreiben, und des ersten Satzes von Sensordaten, der aus einem Speicher (115) des tragbaren Geräts abgerufen wird;
Anweisungen zum Abfragen des Sensors (130, 135, 140, 145, 150) nach neuen Sensordaten, um einen zweiten Satz von Sensordaten zu erhalten;
Anweisungen zum Vergleichen des zweiten Satzes von Sensordaten, die vom Sensor (130, 135, 140, 145, 150) erhalten wurden, mit dem Basislinienwert, um festzustellen, ob der zweite Satz von Sensordaten eine Änderung gegenüber dem Basislinienwert darstellt;
Anweisungen zum Anfordern von Benutzereingaben vom Benutzer, die einen aktuellen Kontext beschreiben, als Reaktion auf die Feststellung, dass der zweite Satz von Sensordaten eine Änderung gegenüber dem Ausgangswert darstellt;
Anweisungen zum Erstellen eines Datensatzes basierend auf dem zweiten Satz von Sensordaten und dem aktuellen Kontext; und
Anweisungen zum Speichern des erstellten Datensatzes in einem Speicher (115) als ein Mitglied einer Vielzahl von Datensätzen.

12. Nicht-vorübergehendes computerlesbare Medium nach Anspruch 11, das ferner Anweisungen zur Erkennung des Vorhandenseins eines oder mehrerer anderer tragbarer Geräte umfasst, die kommunikativ mit dem Netzwerk verbunden sind.

13. Nicht-vorübergehendes computerlesbares Medium nach Anspruch 12, das ferner Anweisungen zum Empfang zusätzlicher Kontextdaten von dem einen oder mehreren anderen tragbaren Geräten umfasst, die kommunikativ mit dem Netzwerk verbunden sind, wobei der erstellte Datensatz die zusätzlichen Kontextdaten enthält.

14. Nicht-übergangsweise computerlesbares Medium nach Anspruch 11, wobei die Anweisungen zur Festlegung des Basislinienwertes Anweisungen zur Gewinnung einer statistischen Moduszahl aus dem ersten Satz von Sensordaten umfassen.

15. Nicht-vorübergehendes computerlesbares Medium nach Anspruch 11, das ferner Anweisungen zum Trainieren eines maschinellen Lernmodells unter Verwendung der Mehrzahl von Datensätzen umfasst; sowie Anweisungen zur Anwendung des maschinellen Lernmodells auf einen dritten Satz von Sensordaten, die zu einem späteren Zeitpunkt vom Sensor (130, 135, 140, 145, 150) empfangen werden, um zu einem späteren Zeitpunkt einen Kontext abzuschätzen.

## Revendications

1. Dispositif portable (100) comprenant :
un capteur (130, 135, 140, 145, 150) conçu pour obtenir des données de capteur descriptives d'un paramètre physiologique d'un utilisateur ;
une mémoire (115) conçue pour mémoriser une pluralité d'enregistrements corrélant des données de contexte à des paramètres physiologiques obtenus des données de capteur ; et
un processeur (110) conçu :
pour établir une valeur de référence en fonction d'un premier ensemble de données de capteur obtenues du capteur (130, 135, 140, 145, 150) et récupérées de la mémoire (115),
pour interroger le capteur (130, 135, 140, 145, 150) pour de nouvelles données de capteur pour obtenir un deuxième ensemble de données de capteur,
pour comparer le deuxième ensemble de données de capteur obtenu du capteur (130, 135, 140, 145, 150) à la valeur de référence pour déterminer si le deuxième ensemble de données de capteur est un changement par rapport à la valeur de référence,
pour faire une requête d'une entrée utilisateur d'un descriptif utilisateur d'un contexte actuel en réponse à la détermination que le deuxième ensemble de données de capteur est un changement par rapport à la valeur de référence,
pour créer un enregistrement basé sur le deuxième ensemble de données de capteur et le contexte actuel, et
pour mémoriser l'enregistrement créé dans la mémoire (115) en tant qu'élément de la pluralité d'enregistrements.

2. Dispositif portable (100) selon la revendication 1, comprenant en outre une interface de communication (155) conçue pour communiquer sur un réseau de communication, dans lequel ladite interface de communication (155) est conçue pour détecter la présence d'au moins un autre dispositif portable couplé en communication au réseau.

3. Dispositif portable (100) selon la revendication 2, dans lequel l'interface de communication (155) est en outre conçue pour recevoir des données de contexte supplémentaires provenant de l'au moins un autre dispositif portable couplé en communication au réseau, et dans lequel l'enregistrement créé comprend lesdites données de contexte supplémentaires.

4. Dispositif portable (100) selon la revendication 1, dans lequel le processeur (110) est en outre conçu :
pour apprendre un modèle d'apprentissage automatique à l'aide de la pluralité d'enregistrements ; et
pour appliquer ledit modèle d'apprentissage automatique à un troisième ensemble de données de capteur obtenues ultérieurement du capteur (130, 135, 140, 145, 150) pour estimer ultérieurement un contexte.

5. Dispositif portable (100) selon la revendication 1, dans lequel l'établissement de la valeur de référence comprend l'obtention d'une figure de mode statistique du premier ensemble de données de capteur.

6. Procédé d'apprentissage d'un dispositif portable (100) permettant de prédire un contexte dans lequel un dispositif portable (100) est utilisé, ledit procédé comprenant :
l'établissement d'une valeur de référence en fonction d'un premier ensemble de données de capteur obtenues d'un capteur (130, 135, 140, 145, 150) conçu pour obtenir des données de capteur descriptives d'un paramètre physiologique d'un utilisateur et du premier ensemble de données de capteur récupérées d'une mémoire (115) du dispositif portable ;
l'interrogation du capteur (130, 135, 140, 145, 150) pour de nouvelles données de capteur pour obtenir un deuxième ensemble de données de capteur ;
la comparaison du deuxième ensemble de données de capteur obtenu du capteur (130, 135, 140, 145, 150) à la valeur de référence pour déterminer si le deuxième ensemble de données de capteur est un changement par rapport à la valeur de référence ;
la requête d'une entrée utilisateur du descriptif utilisateur d'un contexte actuel en réponse à la détermination que le deuxième ensemble de données de capteur est un changement par rapport à la valeur de référence ;
la création d'un enregistrement basé sur le deuxième ensemble de données de capteur et le contexte actuel ; et
la mémorisation de l'enregistrement créé dans une mémoire (115) en tant qu'élément d'une pluralité d'enregistrements.

7. Procédé selon la revendication 6, comprenant en outre la détection de la présence d'au moins un autre dispositif portable couplé en communication au réseau.

8. Procédé selon la revendication 7, comprenant en outre la réception de données de contexte supplémentaires provenant de l'au moins un autre dispositif portable couplé en communication au réseau, dans lequel l'enregistrement créé comprend les données de contexte supplémentaires.

9. Procédé selon la revendication 6, dans lequel l'établissement de la valeur de référence comprend l'obtention d'une figure de mode statistique du premier ensemble de données de capteur.

10. Procédé selon la revendication 6, comprenant en outre l'apprentissage d'un modèle d'apprentissage automatique à l'aide de la pluralité d'enregistrements ; et
l'application du modèle d'apprentissage automatique à un troisième ensemble de données de capteur obtenues ultérieurement du capteur (130, 135, 140, 145, 150) pour estimer ultérieurement un contexte.

11. Support lisible par ordinateur non transitoire sur lequel un programme informatique est mémorisé, ledit programme informatique pouvant être exécuté par un processeur (110) pour mettre en œuvre un procédé de prédiction d'un contexte dans lequel un dispositif de santé portable est utilisé, ledit procédé comprenant :
des instructions destinées à l'établissement d'une valeur de référence en fonction d'un premier ensemble de données de capteur obtenues d'un capteur (130, 135, 140, 145, 150) conçu pour obtenir des données de capteur descriptives d'un paramètre physiologique d'un utilisateur et du premier ensemble de données de capteur récupérées d'une mémoire (115) du dispositif portable ;
des instructions destinées à l'interrogation du capteur (130, 135, 140, 145, 150) pour de nouvelles données de capteur pour obtenir un deuxième ensemble de données de capteur ;
des instructions destinées à la comparaison du deuxième ensemble de données de capteur obtenues du capteur (130, 135, 140, 145, 150) à la valeur de référence pour déterminer si le deuxième ensemble de données de capteur est un changement par rapport à la valeur de référence ;
des instructions destinées à la requête d'une entrée utilisateur du descriptif utilisateur d'un contexte actuel en réponse à la détermination que le deuxième ensemble de données de capteur est un changement par rapport à la valeur de référence ;
des instructions destinées à la création d'un enregistrement en fonction du deuxième ensemble de données de capteur et du contexte actuel ; et
des instructions destinées à la mémorisation de l'enregistrement créé dans une mémoire (115) en tant qu'élément d'une pluralité d'enregistrements.

12. Support non transitoire lisible par ordinateur selon la revendication 11, comprenant en outre des instructions destinées à la détection de la présence d'au moins un autre dispositif portable couplé en communication au réseau.

13. Support non transitoire lisible par ordinateur selon la revendication 12, comprenant en outre des instructions destinées à la réception des données de contexte supplémentaires de l'au moins un autre dispositif portable couplé en communication au réseau, dans lequel l'enregistrement créé comprend les données de contexte supplémentaires.

14. Support non transitoire lisible par ordinateur selon la revendication 11, dans lequel les instructions destinées à l'établissement de la valeur de référence comprennent des instructions pour obtenir une figure de mode statistique provenant du premier ensemble de données de capteur.

15. Support non transitoire lisible par ordinateur selon la revendication 11, comprenant en outre des instructions destinées à l'apprentissage d'un modèle d'apprentissage automatique à l'aide de la pluralité d'enregistrements ; et
des instructions destinées à l'application du modèle d'apprentissage automatique à un troisième ensemble de données de capteur obtenues ultérieurement du capteur (130, 135, 140, 145, 150) pour estimer ultérieurement un contexte.
